(19) Europäisches Patentamt
European Patent Office
Office européen des brevets

(11) **EP 2 897 597 B1**

(12) # EUROPEAN PATENT SPECIFICATION

(45) Date of publication and mention
of the grant of the patent:
**08.07.2020 Bulletin 2020/28**

(21) Application number: **13839275.8**

(22) Date of filing: **20.09.2013**

(51) Int Cl.:
*A61K 9/58* (2006.01)       *A61K 9/50* (2006.01)
*A61K 31/542* (2006.01)     *A61K 31/433* (2006.01)

(86) International application number:
**PCT/US2013/060987**

(87) International publication number:
**WO 2014/047477 (27.03.2014 Gazette 2014/13)**

(54) **MULTILAYER BIODEGRADABLE MICROPARTICLES FOR SUSTAINED RELEASE OF THERAPEUTIC AGENTS**

MEHRSCHICHTIGE, BIOLOGISCH ABBAUBARE MIKROPARTIKEL ZUR VERZÖGERTEN FREISETZUNG VON THERAPEUTIKA

MICROPARTICULES BIODÉGRADABLES MULTI-COUCHES POUR UNE LIBÉRATION PROLONGÉE D'AGENTS THÉRAPEUTIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.09.2012 US 201261703743 P**

(43) Date of publication of application:
**29.07.2015 Bulletin 2015/31**

(73) Proprietor: **OHR Pharma, LLC
New York, NY 10022 (US)**

(72) Inventors:
- **RHODES, Chris
  Great Neck, New York 11020 (US)**
- **MALAVIA, Nikita
  Great Neck, New York 11020 (US)**
- **JENNINGS, Robert
  Great Neck, New York 11020 (US)**
- **LAXMA, Reddy
  Great Neck, New York 11020 (US)**
- **NORMAN, Betty
  Great Neck, New York 11020 (US)**

(74) Representative: **Murgitroyd & Company
Murgitroyd House
165-169 Scotland Street
Glasgow G5 8PL (GB)**

(56) References cited:
**US-A1- 2004 115 279      US-A1- 2009 136 583
US-A1- 2011 256 218      US-A1- 2011 256 218
US-A1- 2012 027 833**

- **ACHARYA G ET AL: "The hydrogel template method for fabrication of homogeneous nano/microparticles", JOURNAL OF CONTROLLED RELEASE, ELSEVIER, AMSTERDAM, NL, vol. 141, no. 3, 15 February 2010 (2010-02-15), pages 314-319, XP026883341, ISSN: 0168-3659, DOI: 10.1016/J.JCONREL.2009.09.032 [retrieved on 2009-10-12]**
- **S RAVI ET AL: "Development and characterization of polymeric microspheres for controlled release protein loaded drug delivery system", INDIAN JOURNAL OF PHARMACEUTICAL SCIENCES, vol. 70, no. 3, 1 January 2008 (2008-01-01), page 303, XP055179745, ISSN: 0250-474X, DOI: 10.4103/0250-474X.42978**
- **ALRASHIDI ET AL.: 'Fluidised bed granule coating: Case studies of top and bottom spray coating.' GLATT TIMES. no. 30, 2010 - 26 March 2014, pages 10 - 12, XP055239844 Retrieved from the Internet: <URL:http://www.glatt.com/cm/fileadmin/material/glatt_times_30.pdf>**

## Description

## TECHNICAL FIELD

This invention relates to methods for forming multilayer microparticles for sustained release of therapeutic agents and compositions comprising multilayer microparticles.

## BACKGROUND

Microparticles composed of a biodegradable polymer are useful for controlled release of therapeutic agents. Microparticles can be formed using a template (US 2009/0136583). Acharya et al., Journal of Controlled Release, vol. 141, no. 3, 15 February 2010, pages 314-319, discloses a method for preparing microparticles with a homogenous size distribution using a gelatin hydrogel template comprising circular wells filled with PLGA solution.

## SUMMARY

The present disclosure features methods of forming multilayer microparticles for sustained release of therapeutic agents. These methods include (a) forming a layer comprising a first polymer on a solid planar surface that is the base of a well by depositing a first composition one or more times on the solid surface, wherein the first composition comprises the first polymer and a first solvent, and evaporating the first solvent in the first composition; (b) forming one or more layers comprising a second polymer and a therapeutic agent by depositing a second composition on all or part of the layer formed in step (a), wherein the second composition comprises the second polymer, the therapeutic agent, and a second solvent; and evaporating the second solvent in the second composition; and (c) forming an additional layer comprising a third polymer by depositing a third composition one or more times on a previously formed layer, wherein the third composition comprises the third polymer and a third solvent; and evaporating the third solvent in the third composition. The average molecular weight of the first and third polymers is greater than the average molecular weight of the second polymer by at least 40 kilodalton. The first, second, and third polymers are poly(lactic-co-glycolic acid) (PLGA). In some cases the first and third polymers have the same molecular weight and in some cases they differ in molecular weight. In some cases there are two or more inner layers. For example, one inner layer can contain a first therapeutic agent and the second inner layer can contain a second therapeutic agent. In addition, when there are two or more inner layers they can differ in the polymer type or molecular weight. In addition, where there are two or more inner layers, they can be formed using compositions that differ in solvent, As explained in greater detail below, when adjacent layers are formed using compositions that differ with respect to solvent and/or polymer, there is less tendency for material from the two adjacent layers to intermingle during layer formation. The first and the third compositions do not contain a therapeutic agent, thus the layers formed by the first and the third compositions contain only polymer or only polymer and non-therapeutic components. The polymer outer layers can act as barriers in controlling the initial burst release of the therapeutic agent and further reduce its subsequent release rate from the inner layer of the microparticle. Thus, the methods of forming microparticles disclosed herein are useful for achieving sustained release of therapeutic agents with reduced or no burst release.

Described herein is a method for preparing a multilayer microparticle as claimed in claim 1, the method comprising:

(a) forming a layer comprising a first polymer on a solid planar surface that is the base of a well by depositing a first composition one or more times on the solid surface, wherein the first composition comprises the first polymer and a first solvent, and evaporating the first solvent in the deposited first composition;

(b) forming a layer comprising a second polymer and a therapeutic agent by depositing a second composition on all or part of the layer formed in step (a), wherein the second composition comprises the second polymer, the therapeutic agent, and a second solvent; and evaporating the second solvent in the deposited second composition; and

(c) forming an additional layer comprising a third polymer by depositing a third composition one or more times on a previously formed layer, wherein the third composition comprises the third polymer and a third solvent; and evaporating the third solvent in the deposited third composition;

wherein the first and the third compositions do not contain a therapeutic agent; wherein the first, second, and third polymers are poly(lactic-co-glycolic acid) (PLGA), and wherein the average molecular weight of the first and the third polymers is greater than the average molecular weight of the second polymer by at least 40 kilodalton.

In various embodiments: the first and the third polymers have low solubility in the second solvent; the average molecular weight of the first and the third polymers is greater than the average molecular weight of the second polymer by at least 50 kilodalton; the first and the third polymers have an average molecular weight of 100-350 kilodalton; the second polymer has an average molecular weight of 15-150 kilodalton; the first and the third solvents are the same solvent; the second solvent differs from the first and third solvents; the therapeutic agent is selected from the group consisting of a small molecule drug, a peptide drug, a protein drug, a polysaccharide drug, an oligonucleotide, and an antibody; step (a) comprises depositing the first composition more than once; step (a) comprises depositing the first composition twice and evaporating the first solvent in the first composition twice; step (c) comprises depositing the third composition more than once; step (c) comprises by dispensing the third composition and evaporating the third solvent in the third composition twice; the solid surface is coated; wherein the well can be partially filled, completely fill or over filled.

In some cases: the depositing comprises spraying using a device (e.g., a microprinter or spray jet printer) that generates droplets having an average diameter less than 200, 150, 100 or 60 microns.

Also disclosed, but not claimed, is a composition comprising one or more multilayer microparticles, wherein the one or more multilayer microparticles comprise:

a first layer comprising a first polymer; and a second layer comprising a therapeutic agent and a second polymer, and a third layer comprising a third polymer.
wherein the molecular weights of the first and third polymers are greater than the molecular weight of the second polymer.

In various embodiments of the composition disclosed, but not claimed: the first and third (also called top and bottom) layers do not contain a therapeutic agent; the molecular weight of the first polymer and the third polymer is greater than the molecular weight of the second polymer by at least 20 kilodalton; the first and second polymers have a molecular weight of 100-350 kilodalton; the second polymer has a molecular weight of 15-150 kilodalton; the polymers are selected from the group consisting of poly(lactic acid) (PLA), poly(L-lactic acid) (PLLA), poly(glycolic acid) (PGA), poly($\varepsilon$-caprolactone), and poly(ortho ester); the therapeutic agent is selected from the group consisting of a small molecule drug, a peptide drug, a protein drug, a polysaccharide drug, an oligonucleotide, and an antibody; the multilayer microparticles are essentially symmetrical in three dimensions and no one dimension is greater than 80 microns; the multilayer microparticles are symmetrical in two dimensions wherein the dimension along the longer axis of symmetry is less than 100 microns, and the dimension along the shorter axis of symmetry is less than 60 microns; the composition is an implant with a greatest linear dimension that is less than 10 mm; the composition is an implant with a greatest linear dimension that is less than 2 mm; the composition is an implant with a greatest linear dimension that is less than 500 microns; the composition further comprising a pharmaceutically acceptable carrier or excipient; the multilayer microparticles comprise three or more layers; the multilayer microparticles comprise five or more layers; the multilayer microparticles comprise layers of uniform thickness; the multilayer microparticles comprise layers of different thickness; the multilayer microparticles comprise one or more layers not coincident with an adjacent layer; the multilayer microparticles comprise one or more layers with an opening (e.g., a ring-shaped opening); and the micorparticles have two opposing substantially parallel surfaces; and the particles are substantially cylindrical.

As described herein, a particle can have multiple layers and each layer can be formed by multiple applications of a given composition. However, even if two or more depositions of a composition are required to form a layer, the layer is still considered a single layer because the same composition was used for each deposition used to form the layer.

In all embodiments, the polymer used to form the various layers is a biodegradable polymer, for example, a pharmaceutically acceptable biodegradable polymer for administration to a human, for example, the eye of a human.

The details of one or more embodiments of the invention are set forth in the accompanying drawings and the description below. Other features, objects, and advantages of the invention will be apparent from the description and drawings, and from the claims.

## DESCRIPTION OF DRAWINGS

FIG. 1 is a line graph showing that an outer layer of 178 kilodalton (kDa) PLGA reduced the initial burst release and subsequent release rate of brinzolamide from the brinzolamide-containing microparticles in an *in vitro* drug release study.
FIG. 2 is a line graph showing that an outer layer of 180 kDa PLLA-20 reduced the initial burst release and subsequent release rate of brinzolamide from the brinzolamide-containing microparticles to a similar extent as the outer layer of 178 kDa PLGA did.
FIG. 3 is a line graph showing that an outer layer of 109 kDa PLGA reduced the initial burst release of acetazolamide from the acetazolamide-containing microparticles in an in vitro drug release study.

## DETAILED DESCRIPTION

The present disclosure features methods of forming multilayer microparticles for sustained release of therapeutic agents. The multilayer microparticles can be formed using the following steps. First, a bottom outer layer comprising a first polymer can be formed by depositing a first composition, which contains the first polymer and a first solvent, one or more times on a solid surface and evaporating the first solvent. Next, one or more inner layers can be formed by depositing a second composition, which contains a second polymer, a therapeutic agent, and a second solvent, on all or part of the bottom layer and evaporating the second solvent. Finally, an additional top outer layer comprising a third polymer can be formed by depositing a third composition, which comprises a third polymer and a third solvent, one or more times on the last formed layer and evaporating the third solvent. In some embodiments, the first and the third compositions are the same composition. For example, the first and the third solvents can be the same solvent. In some cases the first and third solvents differ from the second solvent such that the first and third polymers are less soluble in the second solvent than in the second polymer.

The first and the third compositions do not contain a therapeutic agent, thus, the top and bottom outer layers formed by the first and the third compositions are polymer alone outer layers or contain polymer and non-therapeutic components. Drug-polymer microparticles without such outer layers tend to have drug pockets which are caused by a physical separation between the drug and the polymer during the solvent evaporation step, possibly due to the differential solubility of the drug and polymer in the solvent, and the migration of the drug along with the evaporating solvent towards the microparticle surface. After administration, the drug on the microparticle surface, especially from the drug pockets, tends to release as a large initial burst. Moreover, the dissolution of drug pockets results in more porous microparticles and this further enhances the drug release rate. The microparticles disclosed herein have outer layers which can act as barriers in controlling the initial burst release of the therapeutic agent contained in the inner layer or layers and can lower the subsequent release rate from the inner layers of the microparticle. Thus, the methods of forming microparticles disclosed herein are useful for achieving sustained release of therapeutic agents over an extended duration.

In some embodiments disclosed, but not claimed, the outer layers (sometimes referred to as the top and bottom layers) can contain a therapeutic agent and this therapeutic agent can be the same therapeutic agent as present in the inner layer (or layers) or can be different,

To avoid partial dissolution of a previously formed layer in a subsequent deposition step and to avoid intermingling of adjacent layers, in some embodiments, the polymer in the previously formed layer can have low solubility in the solvent of a subsequently applied composition. For example, the first polymer can have low solubility in the second solvent, and thus the dried bottom outer layer comprising the first polymer is not significantly solubilized by the second solvent during the formation of the therapeutic agent-containing inner layer or layers. The third polymer can also have low solubility in the second solvent, and thus the additional top outer layer comprising the third polymer is not mixed with the last therapeutic agent-containing inner layer because the second polymer and the third polymer stay phase separated due to their differential solubility in the second solvent.

In some cases, the solvent used in the compositions used to form adjacent layers is the same, but the difference in polymer molecular weight reduced the tendency of a subsequently applied solvent containing composition to dissolve or partially dissolve previously formed layer.

The solubility of a polymer in a given solvent can be estimated by the Hilderbrand solubility parameter ($\delta$). The calculation of Hilderbrand solubility parameter ($\delta$) is described in the review article authored by Miller-Chou, B.A. and Koenig, J.L. (A review of polymer dissolution, Prog. Polym. Sci. 28:1223-1270, 2003). Briefly, Hilderbrand solubility parameter ($\delta$) is the square root of the cohesive energy density (CED):

$$\delta = (\text{CED})^{1/2} = (E/V)^{1/2} = [(\Delta H_{vap} - RT)/V]^{1/2}$$

where $\Delta H_{vap}$ is the enthalpy of vaporization, V is the volume, and T is the absolute temperature. Solubility is largely affected by the structural similarity between the polymer and the solvent, which is known as the "like dissolves like" principle. Thus, if $\delta 1$ is the Hilderbrand solubility parameter of the solvent, and $\delta 2$ is the Hilderbrand solubility parameter of the polymer, a polymer shows high solubility in a solvent when $|\delta 1 - \delta 2|$ is small, e.g., $|\delta 1 - \delta 2| < 4$. To the contrary, when $|\delta 1 - \delta 2|$ is large, e.g., $|\delta 1 - \delta 2| > 4$, a polymer has low solubility in the solvent. In some embodiments, the second solvent is selected based on the therapeutic agent in the second composition. The first and the third polymers can be selected based on a large difference between its Hilderbrand solubility parameter and that of the second solvent (i.e., large $|\delta 1 - \delta 2|$ value).

The molecular weight of a polymer affects its solubility in a given solvent: higher molecular weight of a polymer lowers its solubility (Prog. Polym. Sci. 28:1223-1270, 2003). The molecular weight of the first and the third polymers is greater than the molecular weight of the second polymer by at least 40 kilodalton (kDa). For example, the molecular weight of the first and the third polymers can be greater than the molecular weight of the second polymer by 40kDa, 45 kDa, 50 kDa, 55 kDa, 60kDa, 65 kDa, 70kDa, 75 kDa, 80 kDa, 85 kDa, 90 kDa, 95 kDa, 100 kDa. For example, the first and the third polymers can have an average molecular weight of 100-350 kDa; and the second polymer can have an average molecular weight of 15-150 kDa.

As disclosed herein, but not claimed, a wide variety of polymers can be used to form the microparticles and the identity and concentration of polymer can vary in the various layers of the microparticles to provide particles with desirable drug release characteristics. Non-limiting examples of polymers include: poly(lactic acid) (PLA), poly(L-lactic acid) (PLLA), poly(glycolic acid) (PGA), poly($\varepsilon$-caprolactone) (PCL), and poly(ortho ester) (POE), and other natural biodegradable polymers, such as collagen, chitosan, and poly(amino acid). In the present invention, the first, second and third polymers are PLGA.

Various therapeutic agents can be delivered using the multilayer microparticles described herein. For example, the therapeutic agent can be a small molecule drug, a peptide drug, a protein drug, a polysaccharide drug, an oligonucleotide, or an antibody.

A variety of solvents can be used in the microparticle fabrication based on the type of the therapeutic agent, the polymer, and the formulation. For example, the first, second, and third solvents can be selected from Class 3 or Class 2 organic solvents according to the ICH Guidance for Industry Q3C Impurities: Residual Solvents issued by the Food and Drug Administration (FDA) in 2012. Class 3 solvents are less toxic and of lower risk to human health and include acetic acid, acetone, anisole, methyl acetate, ethyl acetate, isobutyl acetate, propyl acetate, isopropyl acetate, butyl acetate, 1-butanol, 2-butanol, 3-methyl-1-butanol, methylethylketone, tert-butylmethyl ether, methylisobutylketone, dimethyl sulfoxide (DMSO), 2-methyl-1-propanol, ethanol, ethyl ether, ethyl formate, formic acid, heptane, pentane, 1-pentanol, 1-propanol, and 2-propanol. Class 2 solvents are toxic but can be used in pharmaceutical products if their residual concentration is limited to a FDA specified level. Class 2 solvents include acetonitrile, chlorobenzene, chloroform, cumene, cyclohexane, 1,2-dichloroethene, dicholoromethane, 1,2-dimethoxyethane, N,N-dimethylacetamide, N,N-dimethylformamide, 1,4-dioxane, 2-ethoxyethanol, ethylene glycol, formamide, hexane, methanol, 2-methoxyethanol, methylbutylketone, methylcyclohexane, N-methylpyrrolidone, nitromethane, pyridine, sulfolane, tetrahydrofuran, tetralin, toluene, trichloroethylene, and xylene. These solvents allow great flexibility for various types of therapeutic agent, polymer, and formulation. The second solvent can be selected based on the therapeutic agent of the second composition. The first and the third solvents can be selected based on the first and the third polymers, respectively.

The first, second, and third compositions can be either a liquid, gel or a paste. In some cases the therapeutic agent is at least partially suspended in the composition containing the second solvent and the second polymer rather than fully dissolved. Sometimes a portion of the therapeutic agent is dissolved and a portion is suspended. In some embodiments, the compositions can be deposited onto the solid surface using a device capable of dispensing a small amount of liquid in a controlled manner, e.g., a microprinter. For example, a layer of the microparticle can be formed by the microprinter spraying droplets having an average diameter less than 60 microns onto a solid surface or a previously formed layer. In many cases each layer is formed using one deposition step and one evaporation step. However, in some cases it may be desirable to provide a thicker layer by repeating the deposition and evaporation steps with a chosen composition. For example, the deposition step and the evaporation step can be repeated once, twice, or three times. The solvents can be evaporated by air drying the deposited composition at room temperature, e.g., for five to twenty minutes. The evaporation can occur after each deposition step, after some deposition steps or after all deposition steps for a given layer are complete. However, it preferable the evaporation of substantially all of the solvent in a given layer occurs prior to deposition of material to form the subsequent layer.

Depending on the formulation and solvent composition and deposition process used, microparticles may include a variety of types of layers: 1) simple flat layers that are layered on top of each other, 2) layers that are not coincident on each other, 3) non-uniform layers that have a donut shape where the outer diameter is thicker than a middle portion of the layer, 4) non-uniform layers that have a hemi-spherical shape, where the outer diameter is thinner than a middle portion of the layer. In general, a given layer need not have a uniform thickness.

In some embodiments, the microparticles formed on the planar surface can be subsequently released from the surface as described below. The substantially planar surface can be coated to facilitate deposition of the compositions and/or release of the formed microparticles.

In some embodiments, a template having a plurality of wells can be employed to fabricate the microparticles. Microfabrication techniques employing hydrogel templates are described in: Park (Journal of Controlled Release 141:314-319, 2010). Other micro fabrication techniques employing other types of templates are described in Whitesides (Annual Review Biomed Engineering 3:335-73, 2001). When a template is used, the base of a well in the template serves as the solid surface, and the multilayer microparticles can be formed in one or more wells of the template by completely filling, partially filling, or overfilling the wells with the compositions. Multilayered microparticles formed in this manner will take on the shape of the wells in which they are formed (e.g. cylinders, cubes, rectangular prisms can be formed this way). In addition, nearly spherical particles can be built up by using a hemispherical template or a very low profile template or essentially flat template made of differentially coated glass or other substrate such that the surface of the substrate varies in a manner that allows the deposited composition to retain a particular shape rather than spread across the substrate in an uncontrolled manner. In this manner a hemispherical structure can be produced by building up layers on top of each other until the structure protrudes above the template and is dried. In some cases, the characteristics of the composition (solvent, viscosity, etc.) control the shape of the particle.

When a template having wells is used to form the microparticles, the microparticles are preferably removed from the template by dissolving the template. Thus, the template can be water-soluble, e.g., a hydrogel. Once the microparticles are complete they can be released from the template as described below.

Where a template is used, the template can be formed using a mold. The mold can be prepared by coating a silicon wafer with photoresist and etching out the desired shape for the template, which is then formed on the mold. The wells in the template may be any desired shape such that the resulting microparticles can have at least one cross-section that is square, rectangular, round or some other desired shape.

Microparticles can be released from planar surfaces or templates using any suitable means, such as by immersing in a solvent that does not substantially dissolve the microparticles and filtering or centrifuging. For example, when a water-dissolvable hydrogel template is used, the microparticles can be released by dissolving the templates in water at a desired temperature. The microparticles can be harvested by filtering the microparticle-containing suspension or solution through a sieve, and collecting the microparticles on the top surface of the sieve. To remove excess water, the collected microparticles can be freeze dried, e.g., for 12 hours, and then vacuum dried for one to ten days, e.g., for five days. The support surface or template can be immersed in liquid nitrogen or other cooled gas stream. The microparticles can be blown off with air or under vacuum. Additionally, templates may be immersed in a nonsolvent and sonicated to release microparticles.

Also disclosed herein, but not claimed, are compositions containing multilayer microparticles. Each multilayer microparticle of the composition includes one or more outer layers comprising a first polymer; and one or more inner layers comprising a therapeutic agent and a second polymer. In general, the one or more outer layers do not contain a therapeutic agent. The molecular weight of the first polymer is greater than the molecular weight of the second polymer by at least 20 kilodalton, e.g., by 20 kDa, 25 kDa, 30 kDa, 35 kDa, 40kDa, 45 kDa, 50 kDa, 55 kDa, 60kDa, 65 kDa, 70kDa, 75 kDa, 80 kDa, 85 kDa, 90 kDa, 95 kDa, 100 kDa. For example, the first polymer can have an average molecular weight of 100-350 kDa; the second polymer can have an average molecular weight of 15-150 kDa. The first and second polymers can be selected from: poly(lactic acid) (PLA), poly(L-lactic acid) (PLLA), poly(glycolic acid) (PGA), poly($\varepsilon$-caprolactone) (PCL), and poly(ortho ester) (POE), and other natural biodegradable polymers, such as collagen, chitosan, and poly(amino acid).

In some embodiments, the multilayer microparticles are essentially symmetrical in three dimensions and no one dimension is greater than 80 microns. In some embodiments, the multilayer microparticles are symmetrical in two dimensions, and the dimension along the longer axis of symmetry is less than 100 microns, while the dimension along the shorter axis of symmetry is less than 60 microns.

In some embodiments, the average (on a particle volume basis) Dv (diameter of a spherical particle of the same volume) of the microcapsules is less than 100 $\mu$m; the average Dv of the microcapsules is selected from: less than 90, 80, 70, 60 or 50 $\mu$m. In some embodiments the microparticles are substantially monodiperse. In some embodiments, at least 70% (80%, 90%) of the microcapsules in the composition vary from the average Dv of the microcapsules in the composition by no more than 50% (40%, 30%, 20% or less). In some cases the average greatest linear dimension of the microcapsules is selected from: less than 100, 90, 80, 70, 60, 50 or 40 $\mu$m and is greater than 30, 40 or 50 $\mu$m. In some embodiments, the microparticles comprise three or more layers, e.g., including two outer layers and one inner layer. In some embodiments, the microparticles comprise four or more layers, e.g., including two outer layers (a top outer layer and a bottom outer layer) and two or more (e.g., two, three, four, five, six, seven, eight, nine, or ten) inner layers. The microparticles can include various types of layers. For example, the multilayer microparticles can contain layers of uniform thickness. In some embodiments, the microparticles consist of uniform flat layers that are layered on top of each other. The multilayer microparticles can also contain layers of different thickness. In some embodiments, the microparticles contain one or more layers not coincident with an adjacent layer, e.g., a layer of donut shape with a ring-shaped opening or a hemispherical layer. In general, a given layer need not have a uniform thickness. For example, the microparticles can contain non-uniform layers where the outer diameter is thicker or thinner than the middle portion of the layer.

The compositions described herein, but not claimed, can also include excipients, vehicles, or buffers that are suitable for a particular formulation of the therapeutic agent.

In some embodiments described herein, but not claimed, the compositions containing multilayer microparticles can form an implant when injected into a patient. The implant can have a greatest linear dimension of between 0.5 and 10 mm, e.g., a cylindrical implant with dimensions of 2 mm x 0.75 mm. The total weight of the implant can be 100 to 5000 micrograms (e.g., 250-1000 micrograms). Such a large implant can contain a greater amount of therapeutic agent and the therapeutic agent can be released over a longer period of time. For example, the microparticles can be formulated to release the therapeutic agent over a period of at least 3 months, 6 months, 9 months, 12 months, 18 months, two years or longer.

The compositions can be deposited on a template or a planar surface in a variety of ways. For example, when a microprinter is used for deposition on a planar surface, the area of each layer depends initially on the diameter of the printer nozzle, the amount of liquid composition deposited to form the layer and the physical characteristics of the liquid composition. However, by moving the print head, it is possible to create layers in a variety of sizes and shapes. By using a controllable printhead, different layers can have different sizes and/or shape. For example, a first layer can be a 50 micron diameter disk, the second layer can be a 20 micron diameter disk centered on the first layer and the third layer can be a 50 micron diameter disk centered on the first layer. In some embodiments, the liquid composition can be deposited by a microprinter on a template rather than on a substantially planar surface. In this embodiment, the microprinter deposits the liquid composition in one or more wells of a template having a plurality of wells.

When using a microprinter, the atmosphere of the enclosure in which deposition of the liquid composition takes place can be controlled in order to improve the printing nozzle efficiency, prevent nozzle clogging, control the evaporation of solvent in the deposited liquid composition and to otherwise provide desirable conditions. Depending on the polymer, solvent, therapeutic, excipients, and formulation, it may be useful to increase or decrease the temperature, the humidity, and the atmospheric pressure. It can also be useful to employ an inert gas atmosphere (e.g., nitrogen or argon), or use an atmosphere at least partially saturated with a solvent. Moreover, the temperature of the nozzle and/or the deposition surface can be controlled by cooling or heating.

## EXAMPLES

The invention is further described in the following examples, which do not limit the scope of the invention described in the claims.

Materials

The experiments were performed using commercially available materials: polyvinyl alcohol (PVA, Sigma); poly(lactic-co-glycolic) (PLGA): 54 KDa PLGA (Evonik Industries /Lakeshore Biomaterials 6535DLG4A), 109 or 118 kDa PLGA (Evonik Industries /Lakeshore Biomaterials 8515 DLG 7E), 115 kDa PLGA (Evonik Industries /Lakeshore Biomaterials 7525 DLG 7E), and 178 kDa PLGA (Akina 8520); poly(L-lactic acid) (PLLA): 180 kDa PLLA-20 (Akina); Brinzolamide (BRZ, Chemvon Biotechnology), Acetazolamide (ACZ, Spectrum Chemical), Tetrahydrofuran (THF, EMD Millipore), Dicholoromethane (DCM, EMD Millipore), Dimethylformamide (DMF, EMD Millipore) and Phosphate Buffered Saline pH 7.4 (PBS, VWR International).

Fabrication of silicon wafer master templates by photolithography

A silicon wafer was spin coated with SU8 2010 photoresist (Microchem, MA) at 3,500 rpm for 30 sec to obtain a desired thickness followed by baking at 95 °C for 3 min. The photoresist coated silicon wafer was exposed to UV radiation through a mask containing 10 $\mu$m diameter circular pattern for 12 sec. After exposure, the silicon wafer was post baked at 95 °C for 3 min followed by development in SU-8 developer for 2 min. The silicon wafer was rinsed with isopropanol and dried with nitrogen gas. The wafer thus fabricated contained wells with diameter ranging from 1.5 um to 50 um or larger.

Fabrication of silicon master templates by e-beam lithography

Circular patterns for 500 nm diameter were designed using Auto CAD 2007 program. A 3" silicon wafer (100) covered with 1 $\mu$m thick SiO2 layer (University Wafer) was spin coated with poly(methylmethacrylate) (PMMA, Microchem) photoresist of 300 nm thick layer using a spin coated (SCS P6708 spin coating system, 3500 rpm, 30 sec). The coated PMMA photoresist layer was exposed to electron beam (e-beam) in a preprogrammed pattern using Leica VB6 High Resolution Ultrawide Field Photolithography Instrument (operating at 100 KV, transmission rate 25 MHz current 5 nA). After e-beam lithography, the silicon wafer was developed in 3:1 isopropanol:methyl isobutyl ketone solution to remove exposed regions of the photoresist. A 5 nm chromium layer and 20 nm gold layer were deposited on to this pattern followed by liftoff of the residual PMMA film in refluxing acetone. The pattern was transferred to the underlying silicon oxide by deep reactive ion etching with SF6/O2 plasma. The generated silicon master template was used in the fabrication of hydrogel templates.

Fabrication of dissolvable PVA templates

Temporary templates for producing microcapsules can be formed using polymers that can be dissolved in aqueous solution or in a mixture of aqueous and organic solutions (e.g., water and ethanol). The temperature and or pH of the solution used for template dissolution can be altered, either increased or decreased from the room temperature to dissolve a temporary template. To form the templates used in the Examples, a clear poly(vinyl alcohol) (PVA) solution (15% w/v in water, 5 ml) was transferred with a pipette onto a silicon wafer master template, or an optional intermediate template made of poly(dimethyl siloxane) (PDMS), (3" diameter) containing circular pillars (e.g., of 50 $\mu$m diameter and 70 $\mu$m height). The PVA solution was evenly spread to form a thin film completely covering the master or PDMS intermediate template and kept in an oven at 70 °C for 30 minutes. This step resulted in the formation of a thin and mechanically strong PVA template. The PVA template was peeled away from the master template or PDMS intermediate template. The obtained PVA template was about 3" in diameter, contained circular wells (e.g., of 50 $\mu$m diameter and 70 $\mu$m depth). The PVA template was examined under a bright field reflectance microscope to determine its structural integrity.

Fabrication of therapeutic agent-containing microparticles

The therapeutic agent and a formulation polymer were dissolved in a suitable solvent to make a 10-15% (w/v, sum weight of the therapeutic agent and the polymer) drug-polymer suspension or solution. The therapeutic agent constitutes 1-30% of the total solids; the formulation polymer constitutes the rest of the solids in the drug-polymer suspension/solution. The solvent was selected based on the therapeutic agent. The coating polymer solution was prepared by dissolving a coating polymer in a suitable solvent for that polymer.

For example, to make brinzolamide-containing microparticles, milled or micro fluidized brinzolamide and PLGA (118 kDa or 115 kDa) were dissolved in dicholoromethane to obtain a 15% (w/v) drug-polymer suspension. The coating polymer solution was prepared by dissolving either 178 kDa PLGA (Akina 8520) or 180 kDa PLLA-20 (Akina) in dicholoromethane to reach about 5-7.5% (w/v) concentration.

To make acetazolamide-containing microparticles, acetazolamide was dissolved in dimethylformamide (DMF) first to make a 300 mg/mL stock, and the stock was then homogenized into dicholoromethane anti-solvent to form acetazolamide microcrystals. Acetazolamide microcrystals and PLGA (65kDa) were dissolved in dicholoromethane to obtain a 15% (w/v) drug-polymer suspension. The coating polymer solution was prepared by dissolving 109 kDa PLGA (Evonik Industries /Lakeshore Biomaterials 8515 DLG 7E) in dicholoromethane to reach about 2% (w/v) concentration.

Microparticles were formed using water-soluble PVA hydrogel templates containing circular wells of 50 μm diameter and 70 μm depth. First, a polymer alone bottom outer layer was formed by dispensing 150 μl of the coating polymer solution onto the base of one or more wells in the PVA template. The coating polymer solution can be deposited on the template and a blade can be drawn across the surface of the template to urge the coating polymer solution into the wells and to substantially remove excess solution on the surface of the template between wells. Alternatively, the coating polymer solution can be deposited directly into the wells using a micro dispenser or by spraying. After the coating polymer solution is deposited, the dichloromethane is allowed to evaporate in the air for five minutes at room temperature. The depositing step can be repeated for a thicker outer layer. Thus, the coating polymer solution can be deposited one, two three or more times. However, the wells should be only partially filled during this process. The evaporation of solvent can occur after each depositing step, after fewer than all depositing steps or after all depositing steps for the bottom outer layer have been completed. The solvent in the bottom outer layer should, however, be evaporated before material is deposited to form an inner layer.

Next, a drug containing inner layer was formed by dispensing 150 μl of the drug-polymer suspension onto the previously formed bottom outer layer. The drug-polymer suspension was deposited on the previously formed bottom outer layer followed by evaporation of dichloromethane in the air for five minutes at room temperature. This step was repeated three to six times, a drug-polymer inner layer in the microparticle. The drug-polymer suspension can be deposited in the wells in same manner as the coating polymer solution or in a different manner (e.g., both can be deposited by spreading or one can be deposited by spreading and the other can be deposited by dispensing.

Finally, top outer layer was formed by depostion 150 μl of the coating polymer solution onto the inner layer. The coating polymer solution was evenly spread on the inner layer followed by evaporation of dichloromethane in the air for five minutes at room temperature. Just as with formation of the bottom outer layer, this step can be repeated for a thicker outer layer.

After forming all desired layers, the PVA templates with the microparticles were dried at room temperature for at least 12 hours. Microparticles were then harvested by dissolving the templates in water at 37°C for at least 30 minutes. The microparticle-containing suspension was filtered through a 104 micron sieve first. The filtrate was then filtered by a 45 micron sieve, and the microparticles were collected on the top surface of the 45 micron sieve. The collected microparticles were freeze dried for at least 12 hours and then vacuum dried at 40°C for five days.

*In vitro* drug release study

For the *in vitro* brinzolamide release study, at least 5 mg of brinzolamide-containing microparticles were suspended in 10 mL of phosphate buffered saline (PBS), and placed in a shaking water bath at 37°C for *in vitro* drug release studies. At a designated test point (e.g., every week after the initial incubation), the samples were centrifuged and 1 mL of supernatant was removed for brinzolamide analysis by high-performance liquid chromatography (HPLC). Subsequently, 8 mL of supernatant was removed and discarded, and 9 mL of fresh PBS was added back to the sample. Appropriate corrections were made to account for drug in the 1 mL unremoved supernatant that carries over to the next release period. The same procedure was followed at each time point tested (e.g., one week, two weeks, three weeks, four weeks, five weeks, six weeks, seven weeks, eight weeks, nine weeks, ten weeks, eleven weeks, twelve weeks, thirteen weeks, fourteen weeks, fifteen weeks, sixteen weeks) until a termination point was reached.

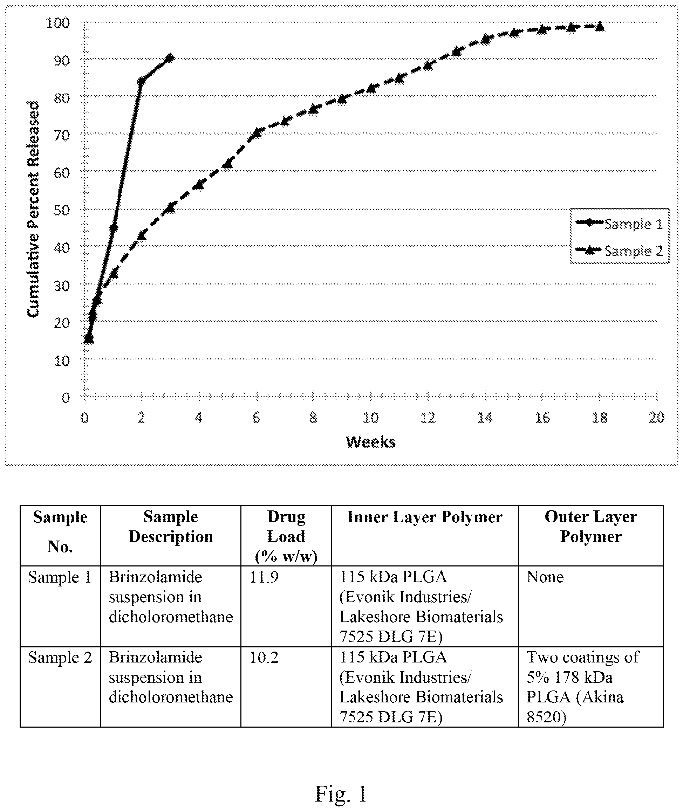

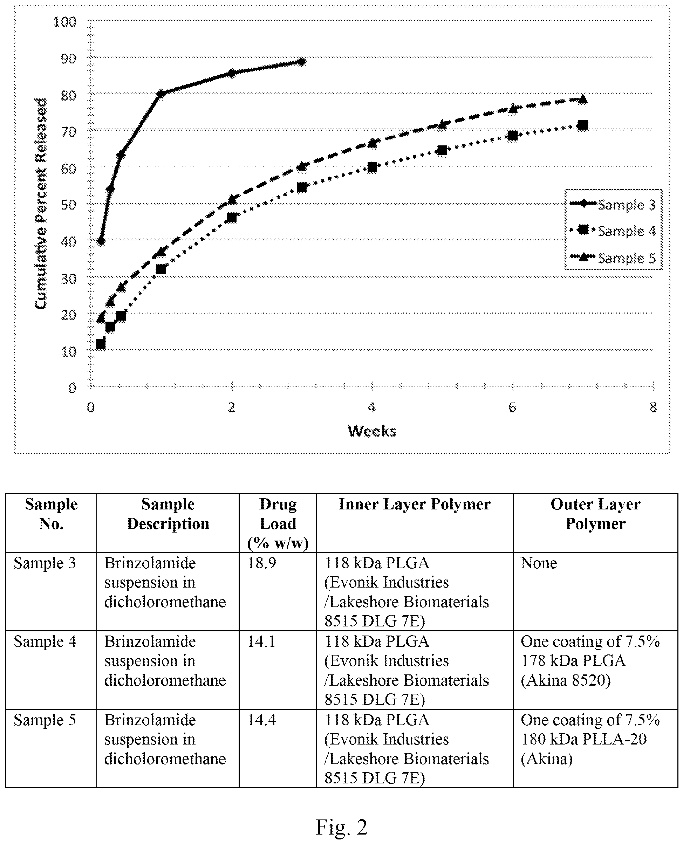

The *in vitro* brinzolamide release results were presented as cumulative percent of the drug released in Figures 1-2. As shown in Figures 1-2, the outer layer of 178 kDa PLGA (Akina 8520) greatly reduced the initial burst release of brinzolamide when compared to the microparticles without such outer layers. Significantly, at two weeks, the cumulative release of brinzolamide from the microparticles with the polymer alone outer layer of 178 kDa PLGA (Akina 8520) is about 40%, while that from the microparticles without the outer layer is more than 80% (Figure 1). In Figure 2, a similar reduction in the initial burst release was observed in microparticles with an outer layer of 180 kDa PLLA-20 (Akina). Moreover, the microparticles with an outer layer of 178 kDa PLGA achieved an extended release of the drug brinzolamide over 18 weeks while similar microparticles without such an outer layer released 90% of the drug during the first three weeks (Figure 1). Similar trend of the extended drug release was observed in the microparticles with an outer layer of 180 kDa PLLA-20 (Figure 2).

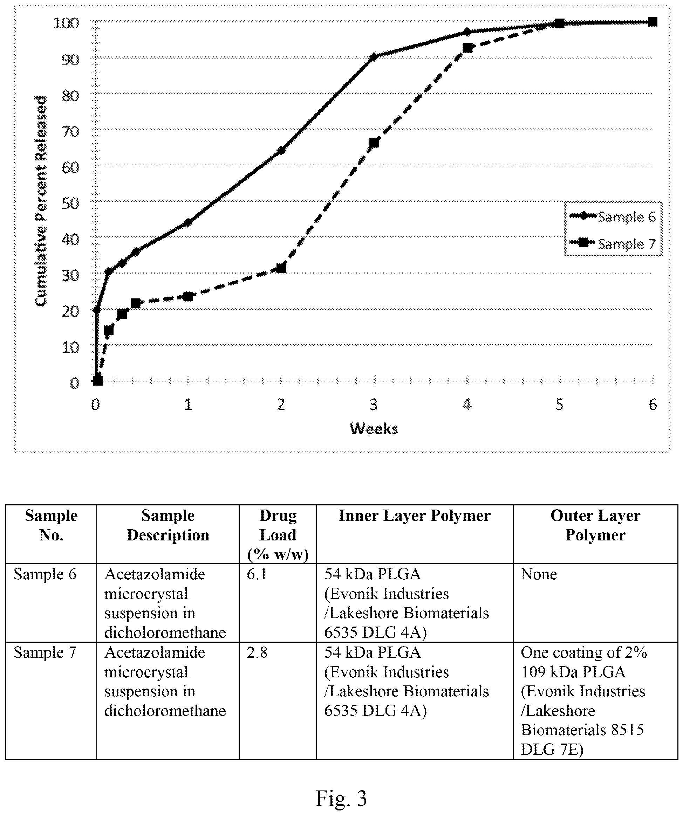

For the in vitro acetazolamide release study, at least 5 mg of acetazolamide-containing microparticles were suspended in 1 mL PBS, and placed in a shaking water bath at 37 °C for *in vitro* drug release studies. At a designated test point (e.g., every week after the initial incubation), the samples were centrifuged and 0.9 mL of supernatant was removed for acetazolamide analysis by HPLC. Subsequently, 0.9 mL of fresh PBS was added back to the sample. Appropriate corrections were made to account for acetazolamide in the unremoved 0.1 mL of solution that carries over to the next release period. This procedure was followed at each time point until a termination point was reached. The *in vitro* acetazolamide release results were presented as cumulative percent of the drug released in Figure 3. As Figure 3 shows, a modest 2% 109kDa PLGA outer layer has also reduced the initial burst release of acetazolamide from the acetazolamide-containing microparticles.

OTHER EMBODIMENTS

It is to be understood that while the invention has been described in conjunction with the detailed description thereof, the foregoing description is intended to illustrate and not limit the scope of the invention, which is defined by the scope of the appended claims.

**Claims**

1. A method for preparing a multilayer microparticle, the method comprising:

    (a) forming a layer comprising a first polymer on a solid planar surface that is the base of a well by depositing a first composition one or more times on the solid surface, wherein the first composition comprises the first polymer and a first solvent, and evaporating the first solvent in the deposited first composition;
    (b) forming a layer comprising a second polymer and a therapeutic agent by depositing a second composition on all or part of the layer formed in step (a), wherein the second composition comprises the second polymer, the therapeutic agent, and a second solvent; and evaporating the second solvent in the deposited second composition; and
    (c) forming an additional layer comprising a third polymer by depositing a third composition one or more times on a previously formed layer, wherein the third composition comprises the third polymer and a third solvent; and evaporating the third solvent in the deposited third composition; wherein the first and the third compositions do not contain a therapeutic agent; wherein the first, second, and third polymers are poly(lactic-co-glycolic acid) (PLGA), and wherein the average molecular weight of the first and the third polymers is greater than the average molecular weight of the second polymer by at least 40 kilodalton.

2. The method of claim 1, wherein the average molecular weight of the first and the third polymers is greater than the average molecular weight of the second polymer by at least 50 kilodalton.

3. The method of claim 1, wherein the first and the third polymers have an average molecular weight of 100-350 kilodalton.

4. The method of claim 1, wherein the second polymer has an average molecular weight of 15-150 kilodalton.

5. The method of claim 1, wherein the first and the third solvents are the same solvent.

6. The method of claim 1, wherein the second solvent differs from the first and third solvents.

7. The method of claim 1, wherein step (a) comprises depositing the first composition more than once.

**Patentansprüche**

1. Verfahren zum Zubereiten eines mehrschichtigen Mikropartikels, wobei das Verfahren Folgendes beinhaltet:

    (a) Bilden einer Schicht, beinhaltend ein erstes Polymer, auf einer festen ebenen Oberfläche, die die Basis einer Vertiefung ist, durch Abscheiden einer ersten Zusammensetzung ein oder mehrere Male auf der festen Oberfläche, wobei die erste Zusammensetzung das erste Polymer und ein erstes Lösemittel beinhaltet; und Verdampfen des ersten Lösemittels in der abgeschiedenen ersten Zusammensetzung;
    (b) Bilden einer Schicht, beinhaltend ein zweites Polymer und ein therapeutisches Mittel durch Abscheiden einer zweiten Zusammensetzung auf der gesamten oder eines Teils der in Schritt (a) gebildeten Schicht, wobei die zweite Zusammensetzung das zweite Polymer, das therapeutische Mittel und ein zweites Lösemittel beinhaltet; und Verdampfen des zweiten Lösemittels in der abgeschiedenen zweiten Zusammensetzung; und

(c) Bilden einer zusätzlichen Schicht, beinhaltend ein drittes Polymer, durch Abscheiden einer dritten Zusammensetzung ein oder mehrere Male auf einer vorher gebildeten Schicht, wobei die dritte Zusammensetzung das dritte Polymer und ein drittes Lösemittel beinhaltet; und Verdampfen des dritten Lösemittels in der abgeschiedenen dritten Zusammensetzung;

wobei die erste und die dritte Zusammensetzung kein therapeutisches Mittel enthalten; wobei das erste, zweite und dritte Polymer Poly(milch-co-glycolsäure) (PLGA) sind und wobei das durchschnittliche Molekulargewicht des ersten und des dritten Polymers um mindestens 40 Kilodalton größer ist als das durchschnittliche Molekulargewicht des zweiten Polymers.

2. Verfahren gemäß Anspruch 1, wobei das durchschnittliche Molekulargewicht des ersten und des dritten Polymers um mindestens 50 Kilodalton größer ist als das durchschnittliche Molekulargewicht des zweiten Polymers.

3. Verfahren gemäß Anspruch 1, wobei das erste und das dritte Polymer ein durchschnittliches Molekulargewicht von 100-350 Kilodalton aufweisen.

4. Verfahren gemäß Anspruch 1, wobei das zweite Polymer ein durchschnittliches Molekulargewicht von 15-150 Kilodalton aufweist.

5. Verfahren gemäß Anspruch 1, wobei das erste und das dritte Lösemittel das gleiche Lösemittel sind.

6. Verfahren gemäß Anspruch 1, wobei sich das zweite Lösemittel von dem ersten und dritten Lösemittel unterscheidet.

7. Verfahren gemäß Anspruch 1, wobei Schritt (a) das Abscheiden der ersten Zusammensetzung mehr als einmal beinhaltet.

**Revendications**

1. Un procédé pour préparer une microparticule multicouche, le procédé comprenant :

(a) la formation d'une couche comprenant un premier polymère sur une surface plane solide qui est la base d'un puits par dépôt d'une première composition une ou plusieurs fois sur la surface solide, la première composition comprenant le premier polymère et un premier solvant, et évaporation du premier solvant dans la première composition déposée ;

(b) la formation d'une couche comprenant un deuxième polymère et un agent thérapeutique par dépôt d'une deuxième composition sur toute ou partie de la couche formée dans l'étape (a), la deuxième composition comprenant le deuxième polymère, l'agent thérapeutique, et un deuxième solvant ; et évaporation du deuxième solvant dans la deuxième composition déposée ; et

(c) la formation d'une couche additionnelle comprenant un troisième polymère par dépôt d'une troisième composition une ou plusieurs fois sur une couche formée précédemment, la troisième composition comprenant le troisième polymère et un troisième solvant ; et évaporation du troisième solvant dans la troisième composition déposée ;

dans lequel les première et troisième compositions ne contiennent pas d'agent thérapeutique ;
dans lequel les premier, deuxième, et troisième polymères sont un poly(acide lactique-co-glycolique) (PLGA), et dans lequel la masse moléculaire moyenne des premier et troisième polymères est supérieure à la masse moléculaire moyenne du deuxième polymère d'au moins 40 kilodaltons.

2. Le procédé de la revendication 1, dans lequel la masse moléculaire moyenne des premier et troisième polymères est supérieure à la masse moléculaire moyenne du deuxième polymère d'au moins 50 kilodaltons.

3. Le procédé de la revendication 1, dans lequel les premier et troisième polymères ont une masse moléculaire moyenne de 100 à 350 kilodaltons.

4. Le procédé de la revendication 1, dans lequel le deuxième polymère a une masse moléculaire moyenne de 15 à 150 kilodaltons.

5. Le procédé de la revendication 1, dans lequel les premier et troisième solvants sont le même solvant.

6. Le procédé de la revendication 1, dans lequel le deuxième solvant est différent des premier et troisième solvants.

7. Le procédé de la revendication 1, dans lequel l'étape (a) comprend le dépôt de la première composition plus d'une fois.

| Sample No. | Sample Description | Drug Load (% w/w) | Inner Layer Polymer | Outer Layer Polymer |
|---|---|---|---|---|
| Sample 1 | Brinzolamide suspension in dicholoromethane | 11.9 | 115 kDa PLGA (Evonik Industries/ Lakeshore Biomaterials 7525 DLG 7E) | None |
| Sample 2 | Brinzolamide suspension in dicholoromethane | 10.2 | 115 kDa PLGA (Evonik Industries/ Lakeshore Biomaterials 7525 DLG 7E) | Two coatings of 5% 178 kDa PLGA (Akina 8520) |

Fig. 1

| Sample No. | Sample Description | Drug Load (% w/w) | Inner Layer Polymer | Outer Layer Polymer |
|---|---|---|---|---|
| Sample 3 | Brinzolamide suspension in dicholoromethane | 18.9 | 118 kDa PLGA (Evonik Industries /Lakeshore Biomaterials 8515 DLG 7E) | None |
| Sample 4 | Brinzolamide suspension in dicholoromethane | 14.1 | 118 kDa PLGA (Evonik Industries /Lakeshore Biomaterials 8515 DLG 7E) | One coating of 7.5% 178 kDa PLGA (Akina 8520) |
| Sample 5 | Brinzolamide suspension in dicholoromethane | 14.4 | 118 kDa PLGA (Evonik Industries /Lakeshore Biomaterials 8515 DLG 7E) | One coating of 7.5% 180 kDa PLLA-20 (Akina) |

Fig. 2

| Sample No. | Sample Description | Drug Load (% w/w) | Inner Layer Polymer | Outer Layer Polymer |
|---|---|---|---|---|
| Sample 6 | Acetazolamide microcrystal suspension in dicholoromethane | 6.1 | 54 kDa PLGA (Evonik Industries /Lakeshore Biomaterials 6535 DLG 4A) | None |
| Sample 7 | Acetazolamide microcrystal suspension in dicholoromethane | 2.8 | 54 kDa PLGA (Evonik Industries /Lakeshore Biomaterials 6535 DLG 4A) | One coating of 2% 109 kDa PLGA (Evonik Industries /Lakeshore Biomaterials 8515 DLG 7E) |

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20090136583 A

**Non-patent literature cited in the description**

- **ACHARYA et al.** *Journal of Controlled Release,* 15 February 2010, vol. 141 (3), 314-319
- **MILLER-CHOU, B.A. ; KOENIG, J.L.** A review of polymer dissolution. *Prog. Polym. Sci.,* 2003, vol. 28, 1223-1270
- *Prog. Polym. Sci.,* 2003, vol. 28, 1223-1270
- Q3C Impurities: Residual Solvents. *Food and Drug Administration (FDA),* 2012
- *Journal of Controlled Release,* 2010, vol. 141, 314-319
- *Annual Review Biomed Engineering,* 2001, vol. 3, 335-73